# EUROPEAN PATENT APPLICATION

(11) **EP 3 042 954 A1**
(43) Date of publication of application: **13.07.2016**
(21) Application number: 14841542.5
(22) Date of filing: 05.09.2014
(51) Int. Cl.: C12N 15/09, C07K 1/22, C07K 14/195, C12N 1/15, C12N 1/19, C12N 1/21, C12N 5/10, C12P 21/02

(54) **DISSOCIATION CAPACITY-BOOSTED LIGAND FOR AFFINITY DISSOCIATION MATRIX**

(30) Priority: 06.09.2013 JP 2013184922; 25.10.2013 JP 2013222508
(71) Applicant: Kaneka Corporation, Kita-ku Osaka 530-8288 (JP)
(72) Inventor: YOSHIDA Shinichi, Takasago-shi Hyogo 676-8688 (JP); OSHIMA Kanji, Takasago-shi Hyogo 676-8688 (JP); MINAKUCHI Kazunobu, Takasago-shi Hyogo 676-8688 (JP)
(74) Representative: Hoffmann Eitle
(86) International application number: PCT/JP2014/073518
(87) International publication number: WO 2015/034056

(57) **Abstract**

The present invention produces affinity ligands that make it possible to increase the purity of antibodies themselves, which is the main purpose of affinity purification, and, further, to improve the selectivity for antibody monomers and thereby reduce the load of removing antibody multimers on downstream purification steps. The present invention provides new affinity ligands which are modified proteins obtained by introducing a mutation into a protein to increase the number of acidic amino acids in the amino acid sequence of the protein; methods for designing the modified proteins; methods for producing the modified proteins; and affinity separation matrices containing the modified proteins as ligands immobilized therein.

## Description

### TECHNICAL FIELD

The present invention relates to proteins capable of specifically binding to an antibody, affinity separation matrices containing the proteins as ligands immobilized therein, and methods for separation and purification of antibody derivatives using the matrices.

### BACKGROUND ART

One of important functions of proteins is to specifically bind to a specific molecule. This function plays a key role in immunoreactions or signal transduction in vivo. Meanwhile, techniques using this function for separation and purification of useful substances have also been actively developed. An example of an actual industrial application is Protein A affinity separation matrices that are used to purify (capture) antibody drugs from animal cell cultures at one time at high purity levels.

Most of antibody drugs developed so far are generally monoclonal antibodies, which are produced massively by something such as recombinant cell culture technology. "Monoclonal antibodies" refer to antibodies that are produced by clones of a unique antibody-producing cell. Almost all the antibody drugs currently available on the market are classified into immunoglobulin G (IgG) subclasses based on their molecular structure. Protein A is a cell wall protein produced by the gram-positive bacterium *Staphylococcus aureus,* and contains a signal sequence S, five immunoglobulin-binding domains (E domain, D domain, A domain, B domain, and C domain), and a cell wall-anchoring domain known as XM region (Non Patent Literature 1). In an initial purification step (capture step) in the manufacture of antibody drugs, affinity chromatography columns are commonly used, in which Protein A is immobilized as a ligand on a water-insoluble carrier (Non Patent Literatures 1 and 2).

Various techniques have been developed for improving the performance of Protein A affinity separation matrices. Techniques in ligands have also been developed. Initially, wild-type Protein A was used as a ligand, but Protein A variants recombinantly produced by protein engineering are now used as ligands in many techniques for improving the column performance. In particular, techniques have been developed that modify Protein A to shift the elution pH towards neutral in the process of eluting antibodies adsorbed on a Protein A affinity separation matrix with an acidic solution (Patent Literatures 1 to 5). The techniques allow us to suppress multimer formation of antibodies (aggregations) under acidic conditions.

Since antibody multimers induce side effects or reduction of drug efficacy, it is important to remove antibody multimers in production of antibody drugs. Antibody multimers can be generated not only in the above-described Protein A affinity purification step but in various steps, ranging from cell culture to formulation. Since Protein A affinity separation matrices, although having high binding specificity to antibodies, do not have high ability to separate monomers from multimers, it is common to remove antibody multimers in a more downstream purification process (see Non Patent Literature 3). The above-described Protein A modification techniques (Patent Literatures 1 to 5) are also not intended to improve the ability to separate antibody monomers from multimers.

### CITATION LIST

### PATENT LITERATURE

Patent Literature 1: US 2006/0194950 A1
Patent Literature 2: WO 2010/110288
Patent Literature 3: WO 2011/118699
Patent Literature 4: JP 2010-081866 A
Patent Literature 5: JP 2012-515160 T

### NON PATENT LITERATURE

Non Patent Literature 1: Hober S. et al., "J. Chromatogr. B", 2007, 848, pp. 40-47
Non Patent Literature 2: Low D. et al., "J. Chromatogr. B", 2007, 848, pp. 48-63
Non Patent Literature 3: Vazquez-Rey M. et al., "Biotechnol. Bioeng.", 2011, 108, pp. 1494-1508

### SUMMARY OF INVENTION

### TECHNICAL PROBLEM

In production of antibody drugs, reduction of antibody multimers (aggregates) formed or left through culture, purification, and formulation steps has been a key issue. In order to suppress formation of antibody multimers or to remove antibody multimers, culture, purification, and formulation steps are controlled by complicated procedures and use of additives. Yet, since antibody multimers generated in an upstream of these steps affect the yield and productivity, there is a need for simple, efficient techniques for removing antibody multimers in an upstream process.

An object of the present invention is to produce new ligands for affinity purification that make it possible to increase the purity of antibodies themselves, which is the main purpose of affinity purification, and, further, to improve the selectivity for antibody monomers and remove antibody multimers, thereby reducing the load on subsequent purification steps.

### SOLUTION TO PROBLEM

In order to solve the above problem, the inventors have designed variants of a Protein A IgG-binding domain, which is taken as a protein model, by increasing the number of acidic amino acids in the amino acid sequence of the protein. Based on this, the inventors have completed the present invention. Specifically, the inventors have specified sites in the amino acid sequence of Protein A which do not affect the specific interaction with a target substance (in this case, IgG) of affinity purification and, further, in which when acidic amino acids are introduced therein, the side chain carboxyl groups (cation exchange groups) are likely to affect the non-specific interaction with the target substance of affinity purification. Then, the inventors have designed variants by substituting such amino acid(s) (single residue or multiple residues) by acidic amino acid(s).

That is, the present invention relates to an immunoglobulin G-binding peptide, which is any one of the following peptides (1) to (3):
(1) an immunoglobulin G-binding peptide having an amino acid sequence containing a substitution of at least one amino acid selected from the group consisting of amino acids at positions 7, 18, 39, 40, 46, 54, and 55 in the amino acid sequence of SEQ ID NO:1 or the amino acid sequence of SEQ ID NO:2 with an acidic amino acid;
(2) an immunoglobulin G-binding peptide having an amino acid sequence further containing a substitution, insertion, deletion, and/or addition of at least one amino acid at a position other than positions 7, 18, 39, 40, 46, 54, and 55 in the amino acid sequence of the peptide (1); and
(3) an immunoglobulin G-binding peptide having an amino acid sequence having at least 80% sequence identity to the amino acid sequence of the peptide (1), wherein the acidic amino acid introduced into the peptide (1) by substitution is retained.

Preferably, the acidic amino acid is an acidic amino acid having a carboxyl group in a side chain.

Preferably, the acidic amino acid having a carboxyl group in a side chain is aspartic acid or glutamic acid.

Preferably, the amino acid substituted by the acidic amino acid is at least one amino acid selected from the group consisting of amino acids at positions 7, 18, 40, 46, and 55.

Preferably, the amino acid subjected to substitution, insertion, deletion, and/or addition is at least one amino acid selected from the group consisting of amino acids at positions 1, 2, 3, 4, 6, 23, 29, 33, 35, 36, 37, 42, 49, 50, 57, and 58.

Preferably, the amino acid subjected to substitution, insertion, deletion, and/or addition is at least one amino acid selected from the group consisting of amino acids at positions 29, 33, 36, and 37.

Preferably, at least two amino acids are substituted by acidic amino acids.

Preferably, at least three amino acids are substituted by acidic amino acids.

Preferably, at least four amino acids are substituted by acidic amino acids.

The present invention also relates to an immunoglobulin G-binding peptide, containing multiple domains in which at least two of the immunoglobulin G-binding peptides are connected.

The present invention also relates to a DNA, containing a base sequence encoding the immunoglobulin G-binding peptide.

The present invention also relates to a vector, containing the DNA.

The present invention also relates to a transformant, obtained by introducing the vector into a host cell.

The present invention also relates to an affinity separation matrix, containing the immunoglobulin G-binding peptide immobilized on a water-insoluble carrier.

The present invention also relates to a method for producing immunoglobulin G or an immunoglobulin G derivative, including bringing immunoglobulin G or an immunoglobulin G derivative into contact with the affinity separation matrix, and separating the immunoglobulin G or immunoglobulin G derivative bound to the affinity separation matrix from the affinity separation matrix.

The present invention also relates to a method for producing a modified ligand for affinity purification, including increasing the number of acidic amino acids in an amino acid sequence of a ligand for affinity purification to improve a cation exchange ability of the ligand.

Preferably, the number of acidic amino acids is increased by substitution with an acidic amino acid and/or addition of an acidic amino acid.

Preferably, the target of affinity purification is immunoglobulin G or an immunoglobulin G derivative.

Preferably, the ligand for affinity purification is an immunoglobulin G-binding domain of Protein A, Protein G, or Protein L.

Preferably, the number of acidic amino acids is increased by two or more.

Preferably, the number of acidic amino acids is increased by three or more.

Preferably, the number of acidic amino acids is increased by four or more.

### ADVANTAGEOUS EFFECTS OF INVENTION

In an affinity separation matrix containing a peptide of the present invention as a ligand immobilized on a carrier, the specific binding between the purification target substance and the ligand is weak (absent) under acidic conditions, but the (concerted) interaction with the cation exchange carboxyl group is expected to occur simultaneously (or continuously).

The case where the peptide of the present invention is a Protein A variant is described in detail. Under physiological conditions at neutral pH where an antibody is added to the affinity separation matrix, the antibody is held on the matrix due to the inherently strong specific binding power of Protein A to (the Fc region of) the antibody. The carboxyl group apart from the specific interaction surface with the antibody has a potential to make an electrostatic, non-specific interaction with the antibody, but hardly affects the specific interaction between the antibody and the ligand under physiological conditions at neutral pH. Under acidic conditions where the antibody is eluted, however, as this specific binding is reduced and the antibody is dissociated from the ligand, the electrostatic, non-specific interaction of the carboxyl group in the ligand with the antibody becomes relatively strong. From another standpoint, the carboxyl group can be regarded as a cation exchange group immobilized on the affinity separation matrix via the protein.

Accordingly, in an affinity separation matrix containing a peptide of the present invention as a ligand immobilized on a carrier, the ionic strength-dependent elution behavior is expected to be more significant when the antibody is eluted with an acidic solution. The affinity separation matrix of the present invention can achieve separation of antibody monomers and multimers as observed in cation exchange chromatography (Non Patent Literature 3), thereby making it possible to highly separate monomers from multimers in the affinity purification step.

Also, the affinity separation matrix of the present invention has the following advantages over the conventional methods of separately immobilizing carboxyl and protein ligands on a matrix and the conventional methods of separately mixing a matrix (cation exchange resin) on which carboxyl groups are immobilized and an affinity separation matrix.

That is, with the immunoglobulin G-binding peptide of the present invention, the protein ligand content per unit volume of the matrix is not impaired, unlike the conventional methods, and thus high binding capacity for target substances can be maintained. Further, in the process of producing an affinity separation matrix, while the conventional methods require control of the ratio of two types of ligands or matrices, the use of the immunoglobulin G-binding peptide of the present invention only requires immobilization of the single ligand on a single matrix, which facilitates management of the production process.

### BRIEF DESCRIPTION OF DRAWINGS

Fig. 1 is a view illustrating a method for constructing an expression vector according to Example 2.
Fig. 2 is a view showing overlapped anion exchange chromatographic profiles of various variants according to Example 4.

### DESCRIPTION OF EMBODIMENTS

The present invention relates to an immunoglobulin G-binding peptide, which is any one of the following peptides (1) to (3):
(1) an immunoglobulin G-binding peptide having an amino acid sequence containing a substitution of at least one amino acid selected from the group consisting of amino acids at positions 7, 18, 39, 40, 46, 54, and 55 in the amino acid sequence of SEQ ID NO:1 or the amino acid sequence of SEQ ID NO:2 with an acidic amino acid;
(2) an immunoglobulin G-binding peptide having an amino acid sequence further containing a substitution, insertion, deletion, and/or addition of at least one amino acid at a position other than positions 7, 18, 39, 40, 46, 54, and 55 in the amino acid sequence of the peptide (1); and
(3) an immunoglobulin G-binding peptide having an amino acid sequence having at least 80% sequence identity to the amino acid sequence of the peptide (1), wherein the acidic amino acid introduced into the peptide (1) by substitution is retained.

The amino acid substitutions herein are expressed using the code for the wild-type or non-mutated type amino acid followed by the position number of the substitution and followed by the code for changed amino acid. For example, a substitution of Ala for Gly at position 29 is represented by G29A.

The term "protein" as used herein is intended to include any molecule of polypeptide structure and include fragmented polypeptide chains, and polypeptide chains linked through peptide bonds as well. Thus, the terms "peptide", "polypeptide", and "protein" as used herein have the same meaning. Moreover, the term "domain" refers to a conformational unit of a protein which has a sequence consisting of several tens to hundreds of amino acid residues and sufficiently fulfills a certain physicochemical or biochemical function. Moreover, a protein containing at least one amino acid substitution, addition, insertion, or deletion relative to the wild-type protein sequence is referred to as a "variant".

One embodiment of the present invention includes use of a protein having affinity for a certain substance as an affinity ligand. Similarly, another embodiment of the present invention includes an affinity separation matrix containing the ligand immobilized on a water-insoluble carrier. The term "affinity ligand" herein refers to a substance (functional group) that selectively captures (binds to) a target molecule among a population of molecules due to a specific affinity between the molecules. Examples of binding due to a specific affinity between molecules include antigen-antibody binding. Examples of affinity ligands include proteins specifically binding to immunoglobulins .

The molecule targeted by the affinity ligands according to the present invention may be any molecule to which the target molecule-binding domains in the affinity ligands can bind, and examples include immunoglobulin G (IgG) and immunoglobulin G derivatives. The term "immunoglobulin G derivative" herein is a generic name of engineered artificial proteins of immunoglobulin G, including, for example, chimeric immunoglobulin Gs in which the domains of human IgG are partially replaced and fused with IgG domains of another biological species, humanized immunoglobulin Gs in which the complementarity determining regions (CDRs) of human IgG are replaced and fused with antibody CDRs of another biological species, immunoglobulin Gs whose Fc region has a molecularly altered sugar chain, and artificial immunoglobulin Gs in which the Fv region and the Fc region of human IgG are fused.

Domains capable of binding to immunoglobulin G or immunoglobulin G derivatives are included in various immunoglobulin-binding proteins. Examples of such immunoglobulin-binding proteins include, but are not limited to, Protein A derived from *Staphylcoccus aureus,* Protein G derived from *Streptococcus* sp. Group C/G, Protein L derived from *Peptostreptococcus magnus,* Protein H derived from group A *Streptococcus,* Protein D derived from *Haemophilus influenzae,* Protein Arp derived from *Streptococcus* AP4, and FcγR of human origin. Preferred is Protein A which is widely used in the initial purification (capture) step for antibody drugs.

The immunoglobulin G (IgG)-binding domains of Protein A include any of E, D, A, B, and C domains of Protein A, and amino acid sequences derived from these domains. These domains have high sequence identity to one another and thus can be aligned. For example, Clustal (http://www.clustal.org/omega/), which is a program for amino acid sequence multiple alignment, may be used for confirmation. As for residue number, the residue at position 31 of the C domain corresponds to that at the same position 31 of the A or B domain, that at position 29 of the E domain, and that at position 34 of the D domain.

The sequences of A to E domains of Protein A are slightly different from each other, but exhibit the same functions. Amino acid sequences before introduction of mutations, which are not the wild-type amino acid sequences of immunoglobulin G-binding domains, exhibit the same functions as the wild-type amino acid sequences if they have high sequence identity to the wild-type amino acid sequences. Even amino acid sequences before mutation which have been altered by partial amino acid substitution, insertion, deletion, or chemical modification are encompassed by the scope of the present invention as long as the proteins having these amino acid sequences have IgG-binding ability. The amino acid sequence before introduction of mutations preferably has at least 80% sequence identity, more preferably at least 85% sequence identity, still more preferably at least 90% sequence identity, even more preferably at least 95% sequence identity, to the wild-type amino acid sequence of the immunoglobulin G-binding domain. The amino acid sequence before introduction of mutations may be the Z domain obtained by introducing the A1V and G29A mutations into the B domain (see Patent Literature 1).

The amino acid sequence of the immunoglobulin G-binding domain of Protein A before introduction of mutations is preferably the amino acid sequence of SEQ ID NO:1 which constitutes the C domain, its variant amino acid sequence of SEQ ID NO:3, or the amino acid sequence of SEQ ID NO:2 which constitutes the Z domain, more preferably the amino acid sequence of SEQ ID NO:1 which constitutes the C domain or its variant amino acid sequence of SEQ ID NO:3. The amino acid sequence before introduction of mutations may also be any other known variant of the immunoglobulin G-binding domain. In particular, preferred are variants obtained by introducing amino acid substitutions at position 29 of the C or Z domain as disclosed in Patent Literatures 1 and 2, and variants obtained by introducing amino acid substitutions at any of positions 31 to 37, preferably position 33, 36, or 37, of the C or Z domain as disclosed in Patent Literature 3.

In the immunoglobulin G-binding peptide (1), the amino acid substituted by an acidic amino acid is preferably at least one amino acid selected from the group consisting of amino acids at positions 7, 18, 40, 46, and 55 in the amino acid sequence of SEQ ID NO:1 or the amino acid sequence of SEQ ID NO:2.

The acidic amino acid is preferably an amino acid having a carboxyl group in a side chain, more preferably aspartic acid or glutamic acid.

In the immunoglobulin G-binding peptide (2), from the standpoint of maintaining the properties of the immunoglobulin G-binding peptide, the number of amino acids to be substituted, inserted, deleted, and/or added is preferably 20 or less, more preferably ten or less, still more preferably five or less, even more preferably four or less, particularly preferably three or less, two or less, or one or less.

However, when acidic amino acids are substituted, inserted, and/or added in the immunoglobulin G-binding peptide (2), from the standpoint of increasing the acidity of the surface of the immunoglobulin G-binding peptide, the number of amino acids to be substituted, inserted, and/or added is preferably one or more, more preferably two or more, still more preferably three or more, even more preferably four or more, particularly preferably five or more.

The amino acid to be substituted, inserted, deleted, and/or added in the immunoglobulin G-binding peptide (2) is preferably at least one amino acid selected from the group consisting of amino acids at positions 1, 2, 3, 4, 6, 23, 29, 33, 35, 36, 37, 42, 49, 50, 57, and 58 in the amino acid sequence of the C domain (SEQ ID NO:1) or the amino acid sequence of the Z domain (SEQ ID NO:2). More preferably, the amino acid to be substituted, inserted, deleted, and/or added is at least one amino acid selected from the group consisting of amino acids at positions 29, 33, 36, and 37 in the amino acid sequence of the C domain (SEQ ID NO:1) or the amino acid sequence of the Z domain (SEQ ID NO:2).

The immunoglobulin G-binding peptide (3) preferably has at least 80% sequence identity, more preferably at least 85% sequence identity, still more preferably at least 90% sequence identity, even more preferably at least 95% sequence identity, to the amino acid sequence of the peptide (1).

The immunoglobulin G-binding peptide (3) has at least 80% sequence identity to the amino acid sequence of the immunoglobulin G-binding peptide (1), provided that the acidic amino acid introduced into the immunoglobulin G-binding peptide (1) is retained without being further altered. The amino acids other than acidic amino acids introduced into the immunoglobulin G-binding peptide (1) may be altered as long as the sequence identity to the amino acid sequence of the immunoglobulin G-binding peptide (1) is at least 80%.

Even one amino acid substitution can be expected to be effective, but the number of amino acid substitutions is preferably two or more, more preferably three or more, still more preferably four or more. The upper limit is not particularly limited, but is preferably nine or less, more preferably seven or less.

Generally, Protein A is a protein containing five connected immunoglobulin-binding domains or immunoglobulin-binding proteins. The immunoglobulin G-binding peptide of the present invention is preferably a multimeric protein (multi-domain protein). The number of single domains in the multimeric protein is preferably two or more, more preferably three or more, still more preferably four or more, even more preferably five or more. The upper limit of the number of single domains connected together is not particularly limited, but is preferably ten or less, more preferably eight or less, still more preferably six or less. Such a multimer may be a homopolymer (e.g. homodimer, homotrimer) consisting of connected immunoglobulin G-binding domains of a single type, or may be a heteropolymer (e.g. heterodimer, heterotrimer) consisting of connected immunoglobulin G-binding domains of different types.

The monomeric proteins obtained by the present invention may be connected without any linker amino acid residues or through one or more amino acid residues, but the connection is not limited to these methods. The number of linker amino acid residues is not particularly limited. Preferably, the monomeric proteins should be connected without destabilizing the three-dimensional structure of the monomeric proteins.

A fusion protein in which the immunoglobulin-binding protein of the present invention or a multimeric protein having two or more of the proteins connected, as a constituent component, is fused with another protein having a different function, is another embodiment of the present invention. Examples of fusion proteins include, but are not limited to, fusion proteins with albumin, glutathione S-transferase (GST), or maltose-binding protein (MBP). Additionally, fusion proteins with a nucleic acid, e.g. a DNA aptamer, a drug, e.g. an antibiotic, or a polymer, e.g. PEG (polyethylene glycol) are also encompassed by the scope of the present invention as long as they take advantage of the benefits of the proteins according to the present invention.

The present invention also relates to a DNA containing a base sequence encoding any of the immunoglobulin G-binding peptides described above. The base sequence may be any base sequence that can be translated into an amino acid sequence forming any of the immunoglobulin G-binding peptides. Such a sequence can be obtained by common known methods, for example, by using a polymerase chain reaction (hereinafter, abbreviated as PCR). It may also be synthesized by known chemical synthesis methods, or is available from DNA libraries. The codons in the base sequence may be replaced with degenerate codons, and the base sequence needs not to be the same as the wild-type base sequence as long as it is translated into the same amino acids as those encoded by the wild-type base sequence. Genetically engineered microorganisms can be obtained using such a DNA, or cell-free protein synthesis systems including such a DNA as a template DNA for transcription may be prepared.

Site-directed mutagenesis for altering the DNA encoding a protein of the present invention can be carried out using recombinant DNA techniques, PCR or the like as follows.

Specifically, mutagenesis by recombinant DNA techniques may be carried out by, for example, cassette mutagenesis in which when there are appropriate restriction enzyme recognition sequences on both sides of a mutagenesis target site in the gene encoding a protein of the present invention, a region containing the mutagenesis target site is removed by cleaving these restriction enzyme recognition sequences with the restriction enzymes, and then a DNA fragment that has been mutated only at the target site by chemical synthesis or the like, is inserted.

Alternatively, site-directed mutagenesis by PCR may be carried out by, for example, a double primer method in which PCR is performed using a double-stranded plasmid encoding the protein as a template, and two types of synthetic oligo primers containing a mutation, complementary to the + and - strands.

Alternatively, a DNA encoding a multimeric protein may be prepared by ligating a desired number of DNAs encoding a monomeric protein (single domain) of the present invention in tandem. For example, the ligation to produce a DNA encoding a multimeric protein may be accomplished by introducing appropriate restriction enzyme sites into the DNA sequence, fragmenting the DNA sequence with the restriction enzymes, and ligating the double-stranded DNA fragments using a DNA ligase. Only one restriction enzyme site may be introduced, or different types of restriction enzyme sites may be introduced. If the base sequences each encoding a monomeric protein in the DNA encoding a multimeric protein are the same, then homologous recombination may be induced in host cells. For this reason, the ligated DNAs encoding a monomeric protein preferably have 90% or lower base sequence identity, more preferably 85% or lower base sequence identity, still more preferably 80% or lower base sequence identity, even more preferably 75% or lower base sequence identity, to one another.

Vectors of the present invention each contain a base sequence encoding any of the proteins described above or a partial amino acid sequence thereof and a promoter that is operably ligated to the base sequence to function in host cells. Typically, such a vector can be constructed by ligating or inserting a gene encoding any of the proteins described above into an appropriate vector. The vector into which the gene is to be inserted may be any vector that is capable of autonomous replication in host cells. The vector may be a plasmid DNA or a phage DNA. For example, when *Escherichia coli* host cells are used, exemplary vectors include pQE vectors (QIAGEN), pET vectors (Merck), and pGEX vectors (GE Healthcare Bio-Sciences). Examples of plasmid vectors useful for gene expression in *Brevibacillus* bacteria include the known *Bacillus subtilis* vector pUB110 and pHY500 (JP H02-31682 A), pNY700 (JP H04-278091 A), pNU211R2L5 (JP H07-170984 A), and pHT210 (JP H06-133782 A), and the shuttle vector pNCMO2 for *Escherichia coli* and *Brevibacillus* bacteria (JP 2002-238569 A).

Transformants of the present invention can be obtained by introducing the recombinant vectors of the present invention into host cells. The recombinant DNAs may be introduced into host cells by, for example, but not limited to, a method using calcium ions, an electroporation method, a spheroplast method, a lithium acetate method, an Agrobacterium infection method, a particle gun method, or a polyethylene glycol method. Moreover, the function of the obtained gene may be expressed in host cells, for example, by incorporating the gene obtained in the present invention into the genome (chromosome). The host cell is not particularly limited, and examples suitable for low-cost mass production are *Escherichia coli, Bacillus subtilis,* and bacteria (eubacteria) of genera including *Brevibacillus, Staphylococcus, Streptococcus, Streptomyces,* and *Corynebacterium*.

The proteins of the present invention may be produced by culturing any of the above-described transformants in a medium to produce and accumulate the protein of the present invention in the cultured cells (including the periplasmic space thereof) or in the culture fluid (extracellularly), and collecting the desired protein from the culture. Alternatively, the proteins of the present invention may be produced by culturing any of the above-described transformants in a medium to produce and accumulate a fusion protein containing the protein of the present invention in the cultured cells (including the periplasmic space thereof) or in the culture fluid (extracellularly), collecting the fusion protein from the culture, cleaving the fusion protein with an appropriate protease, and collecting the desired protein.

The transformants of the present invention can be cultured in a medium according to common methods for culturing host cells. The medium used to culture the obtained transformant may be any medium that allows for high-efficiency, high-yield production of the protein. Specifically, carbon and nitrogen sources such as glucose, sucrose, glycerol, polypeptone, meat extracts, yeast extracts, and casamino acids may be used. In addition, the medium may optionally be supplemented with inorganic salts such as potassium salts, sodium salts, phosphates, magnesium salts, manganese salts, zinc salts, or iron salts. When auxotrophic host cells are used, nutritional substances necessary for their growth may be added. Moreover, antibiotics such as penicillin, erythromycin, chloramphenicol, or neomycin may optionally be added.

Furthermore, in order to inhibit the desired protein from being degraded by intracellular or extracellular proteases derived from host cells, various known protease inhibitors, i.e., phenylmethane sulfonyl fluoride (PMSF), benzamidine, 4-(2-aminoethyl)-benzenesulfonyl fluoride (AEBSF), antipain, chymostatin, leupeptin, pepstatin A, phosphoramidon, aprotinin, and ethylenediaminetetraacetic acid (EDTA), and/or other commercially available protease inhibitors may be added at appropriate levels.

Furthermore, in order to allow the protein of the present invention to be folded accurately, for example, molecular chaperones such as GroEL/ES, Hsp70/DnaK, Hsp90, and Hsp104/ClpB may be used (for example, a molecular chaperone may be coexpressed with the proteins of the present invention or may be allowed to coexist with the protein of the present invention by, for example, combining them into a fusion protein). Other techniques for accurate folding of the proteins of the present invention include, but are not limited to, addition of an additive for assisting accurate folding to the medium; or culturing at low temperatures.

Examples of media that can be used to culture the transformants obtained using *Escherichia coli* host cells include LB medium (1% triptone, 0.5% yeast extract, 1% NaCl) and 2xYT medium (1.6% triptone, 1.0% yeast extract, 0.5% NaCl). Examples of media that can be used to culture the transformants obtained using *Brevibacillus* host cells include TM medium (1% peptone, 0.5% meat extract, 0.2% yeast extract, 1% glucose, pH 7.0) and 2SL medium (4% peptone, 0.5% yeast extract, 2% glucose, pH 7.2).

The proteins of the present invention are accumulated in the cultured cells (including the periplasmic space thereof) or in the culture fluids (extracellularly) by aerobically culturing the cells at a temperature of 15°C to 42°C, preferably 20°C to 37°C, for several hours to several days under aeration and stirring conditions, and then recovered. Optionally, the cells may be cultured anaerobically by blocking the air supply. In the case where the recombinant protein is produced and secreted, the produced recombinant protein can be recovered after culture by separating the cultured cells from the supernatant containing the secreted protein by a common separation method such as centrifugation or filtration. Also, in the case where the protein is accumulated in the cultured cells (including the periplasmic space thereof), the protein produced and accumulated in the cells can be recovered by, for example, collecting the cells from the culture fluid by methods such as centrifugation or filtration, and then disrupting the cells by methods such as sonication or French press, and/or adding something such as a surfactant to make the protein soluble.

Purification of the proteins of the present invention may be accomplished by one or an appropriate combination of techniques such as affinity chromatography, cation or anion exchange chromatography, and gel filtration chromatography. Whether a purified product is a desired protein may be confirmed by common methods, such as SDS polyacrylamide gel electrophoresis, N-terminal amino acid sequencing, or Western blot analysis.

The affinity for an immunoglobulin can be tested using, for example, but not limited to, a biosensor such as a Biacore system (GE Healthcare Bio-Sciences) based on the surface plasmon resonance principle. The measurement conditions may be any conditions in which a binding signal resulting from binding of Protein A to an immunoglobulin can be detected. The affinity can be easily evaluated by measurement at a temperature of 20°C to 40°C (constant temperature) and a neutral pH of 6 to 8.

Examples of binding parameters include the affinity constant (KA) and the dissociation constant (KD) (Nagata et al., "Real-time analysis of biomolecular interactions", Springer-Verlag Tokyo, 1998, p.41). The affinity constant of the proteins of the present invention for Fc can be determined with a Biacore system by adding each domain variant to a flow channel in an experimental system including a sensor chip with human IgG immobilized thereon at a temperature of 25°C and a pH of 7.4. The peptide of the present invention preferably has an affinity constant (KA) for human IgG of at least 1x10⁵ (M⁻¹), more preferably at least 1x10⁶ (M⁻¹), still more preferably at least 1x10⁷ (M⁻¹).

Another embodiment of the present invention includes use of any of the above proteins as an affinity ligand that has affinity for an immunoglobulin derivative. Similarly, another embodiment of the present invention includes an affinity separation matrix containing such a ligand immobilized on a water-insoluble carrier.

Examples of the water-insoluble carrier used in the present invention include inorganic carriers such as glass beads and silica gel; organic carriers such as synthetic polymers (e.g. cross-linked polyvinyl alcohol, cross-linked polyacrylate, cross-linked polyacrylamide, cross-linked polystyrene) and polysaccharides (e.g. crystalline cellulose, cross-linked cellulose, cross-linked agarose, cross-linked dextran); and composite carriers of combinations of these carriers, such as organic-organic or organic-inorganic composite carriers. Examples of commercially available products include GCL2000 (porous cellulose gel), Sephacryl S-1000 (prepared by covalently cross-linking allyldextran with methylenebisacrylamide), Toyopearl (acrylate carrier), Sepharose CL4B (cross-linked agarose carrier) and Cellufine (cross-linked cellulose carrier), although the water-insoluble carrier in the present invention is not limited only to the carriers mentioned above.

In view of the intended purpose and usage of the affinity separation matrix, the water-insoluble carrier used in the present invention desirably has a large surface area, and is preferably a porous matrix having a large number of fine pores of a suitable size. The carrier may be in any form such as beads, monoliths, fibers, or films (including hollow fibers), and any form can be selected.

The ligand may be immobilized on the carrier by conventional coupling methods using, for example, the amino, carboxyl, or thiol group in the ligand. Examples of coupling methods include immobilization methods that involve activation of a carrier by reacting the carrier with cyanogen bromide, epichlorohydrin, diglycidyl ether, tosyl chloride, tresyl chloride, hydrazine, sodium periodate, or the like (or introduction of a reactive functional group into the carrier surface), followed by a coupling reaction between the carrier and a compound to be immobilized as a ligand; and immobilization methods that involve condensation and crosslinking by adding a condensation reagent such as carbodiimide or a reagent containing a plurality of functional groups in the molecule, such as glutaraldehyde, to a system containing a carrier and a compound to be immobilized as a ligand.

A spacer molecule consisting of a plurality of atoms may be introduced between the ligand and the carrier, or alternatively, the ligand may be immobilized directly on the carrier. Accordingly, for immobilization, the proteins of the present invention may be chemically modified or amino acid residues useful in immobilization may be added to the proteins. Examples of amino acids useful in immobilization include amino acids having in side chains functional groups useful in chemical reactions for immobilization, such as Lys containing a side chain amino group or Cys containing a side chain thiol group. The nature of the present invention resides in that the effects provided to the proteins of the present invention are also provided to the matrices in which the proteins are immobilize as ligands. Hence, the proteins modified or altered for immobilization are still encompassed by the scope of the present invention.

The method for producing immunoglobulin G or an immunoglobulin G derivative according to the present invention includes: bringing immunoglobulin G or an immunoglobulin G derivative into contact with the affinity separation matrix, and separating the immunoglobulin G or immunoglobulin G derivative bound to the affinity separation matrix from the affinity separation matrix. Purification of a target molecule with the affinity separation matrix, when the target molecule is adsorbed thereon, can be accomplished according to common affinity column chromatographic purification techniques. In the case where the target molecule is immunoglobulin G, for example, the purification can be accomplished in accordance with affinity column chromatographic purification techniques using a Protein A column which is already commercially available (Non Patent Literature 1). Specifically, a buffer of a protein containing the Fc region of an immunoglobulin is prepared as a neutral solution, and the solution is passed through an affinity column filled with a mixed-mode affinity separation matrix of the present invention to adsorb the protein containing the Fc region of an immunoglobulin. Next, an appropriate amount of buffer allowing the antibody affinity ligand to function is passed through the column to wash the inside of the column. At this time, the desired protein containing the Fc region of an immunoglobulin is adsorbed on the affinity separation matrix of the present invention in the column. Here, impurities can be effectively removed in some cases by optimizing the ionic strength or the composition at a pH near neutral. Conditions where the cation exchange group does not function under a load or during washing are preferred, and in particular, it is preferred to use a washing solution having a high ionic strength in the washing step after adsorption. Subsequently, the cation exchange separation mode is activated by using a combination of an acidic pH and ionic strength, as a result of which highly selective separation can be enabled by the concerted action with the elution from the antibody affinity ligand. It is preferred to reduce ionic strength under neutral conditions before elution at an acidic pH.

In the present invention, the antibody separation function can be controlled by the number (proportion) of substitutions with acidic amino acids in the ligand. For example, in the case where the specific binding capacity of the antibody affinity ligand is higher than the non-specific binding capacity of the cation exchange group, antibodies are eluted from the carrier in acidic elution even at low ionic strength. On the other hand, in the case where the specific binding capacity of the antibody affinity ligand is comparable with or lower than the non-specific binding capacity of the cation exchange group, antibodies eluted from the antibody affinity ligands are transferred to cation exchange group-based binding, so that antibodies are not eluted at low ionic strength. In any case, the elution amount and the proportion of antibody monomers in the eluate are controllable by varying ionic strength. The pH is also a factor that determines elution from the antibody affinity ligand, binding to the cation exchange group, and ionic strength (salt concentration)-dependent elution. In other words, in the affinity separation matrix prepared according to the present invention, separation can be optimized by adjusting elution conditions such as pH and ionic strength. The antibody elution from the affinity separation matrix prepared according to the present invention may be salt concentration gradient or stepwise elution. For the purpose of reducing the amount of the eluate, ionic strength stepwise elution is preferred. Also, for simplification of the operation, conditions that allow collection of antibodies by one-step elution and an increase of monomer content to be achieved are preferred.

The present invention also relates to a method for producing a modified ligand for affinity purification, including increasing the number of acidic amino acids in an amino acid sequence of a ligand for affinity purification to improve the cation exchange ability of the ligand. The acidic amino acid may be an amino acid having a carboxyl group in a side chain and is preferably aspartic acid or glutamic acid. The carboxyl group contained in the acidic amino acid in the ligand can be regarded as a cation exchange group immobilized via the protein on an affinity separation matrix containing the modified ligand for affinity purification immobilized on a carrier. The modified ligand for affinity purification refers to an affinity purification ligand having improvements in affinity for a positively charged purification target and the effect of suppressing adsorption of negatively charged impurities as compared to the affinity purification ligand before the number of acidic amino acids is increased.

The number of acidic amino acids is preferably increased by substitution with an acidic amino acid and/or addition of an acidic amino acid. The number of acidic amino acids is preferably increased by two or more, more preferably by three or more, still more preferably by four or more.

The target of affinity purification is preferably immunoglobulin G or an immunoglobulin G derivative. The ligand for affinity purification is preferably an immunoglobulin G-binding domain of Protein A, Protein G, or Protein L.

### EXAMPLES

The following description is offered to illustrate the present invention in more detail by reference to examples, but the scope of the present invention is not limited to these examples. Proteins obtained in the examples are each represented by "an alphabet indicating a domain - an introduced mutation (wild for the wild type)". For example, the wild-type C domain of Protein A is represented by "C-wild", and a C domain variant containing the G29A mutation is represented by "C-G29A". Variants containing two mutations together are represented by indicating the two mutations together with a slash. For example, a C domain variant containing the G29A and S33E mutations is represented by "C-G29A/S33E". For proteins containing a plurality of single domains connected together, a period (.) and the number of connected domains followed by "d" are further added. For example, a protein consisting of five connected C domain variants containing the G29A and S33E mutations is represented by "C-G29A/S33E.5d".

### (Example 1) Design of C domain variants of Protein A with acidic amino acids introduced therein

In evaluation of sites to which acidic amino acids were to be introduced, the three-dimensional structure of the Z domain (Z-wild.1d, SEQ ID NO:2) was used. Based on the known data on the three-dimensional structure of the Z domain (PDB code: 2SPZ) from a database of protein 3D structures, PDB (http://www.rcsb.org/pdb/home/home.do), the accessible surface area (ASA) of amino acid residues was calculated using DSSP. In order to calibrate the influence of the difference in amino acid side chain size, the rASA (ASA(Xxx)/ASA(Gly-Xxx-Gly), where Xxx is any target amino acid for calculation) was also calculated. The ASA and rASA values of residues are shown in Table 1.

**[Table 1]**

| | Z-Wild.1d (SEQ ID NO:2) | | | C-G29A/S33E.1d (SEQ ID NO:3) | | |
|---|---|---|---|---|---|---|
| Amino acid No. | Amino acid residue | ASA (A²) | rASA | Amino acid residue | ASA (A²) | rASA |
| 1 | V | 115 | 0.72 | A | 96 | 0.85 |
| 2 | D | 66 | 0.44 | D | 81 | 0.54 |
| 3 | N | 170 | 1.08 | N | 160 | 1.01 |
| 4 | K | 61 | 0.29 | K | 65 | 0.31 |
| 5 | F | 30 | 0.14 | F | 47 | 0.22 |
| 6 | N | 64 | 0.41 | N | 84 | 0.53 |
| 7 | K | 129 | 0.61 | K | 151 | 0.72 |
| 8 | E | 41 | 0.22 | E | 30 | 0.16 |
| 9 | Q | 2 | 0.01 | Q | 6 | 0.03 |
| 10 | Q | 18 | 0.10 | Q | 28 | 0.15 |
| 11 | N | 116 | 0.73 | N | 109 | 0.69 |
| 12 | A | 1 | 0.01 | A | 5 | 0.04 |
| 13 | F | 39 | 0.18 | F | 56 | 0.26 |
| 14 | Y | 140 | 0.61 | Y | 137 | 0.60 |
| 15 | E | 55 | 0.30 | E | 50 | 0.27 |
| 16 | I | 0 | 0.00 | I | 0 | 0.00 |
| 17 | L | 84 | 0.47 | L | 80 | 0.44 |
| 18 | H | 125 | 0.64 | H | 129 | 0.66 |
| 19 | L | 4 | 0.02 | L | 4 | 0.02 |
| 20 | P | 108 | 0.76 | P | 101 | 0.71 |
| 21 | N | 52 | 0.33 | N | 59 | 0.37 |
| 22 | L | 3 | 0.02 | L | 0 | 0.00 |
| 23 | N | 110 | 0.70 | T | 100 | 0.68 |
| 24 | E | 61 | 0.33 | E | 54 | 0.30 |
| 25 | E | 133 | 0.73 | E | 138 | 0.75 |
| 26 | Q | 38 | 0.20 | Q | 44 | 0.23 |
| 27 | R | 24 | 0.10 | R | 44 | 0.18 |
| 28 | N | 85 | 0.54 | N | 94 | 0.59 |
| 29 | A | 50 | 0.44 | A | 49 | 0.43 |
| 30 | F | 25 | 0.11 | F | 16 | 0.07 |
| 31 | I | 62 | 0.34 | I | 56 | 0.31 |
| 32 | Q | 90 | 0.48 | Q | 71 | 0.38 |
| 33 | S | 25 | 0.20 | E | 73 | 0.40 |
| 34 | L | 1 | 0.01 | L | 1 | 0.01 |
| 35 | K | 75 | 0.36 | K | 88 | 0.42 |
| 36 | D | 106 | 0.70 | D | 103 | 0.68 |
| 37 | D | 24 | 0.16 | D | 50 | 0.33 |
| 38 | P | 78 | 0.55 | P | 68 | 0.48 |
| 39 | S | 62 | 0.51 | S | 77 | 0.63 |
| 40 | Q | 135 | 0.71 | V | 64 | 0.40 |
| 41 | S | 1 | 0.01 | S | 1 | 0.01 |
| 42 | A | 36 | 0.32 | K | 117 | 0.55 |
| 43 | N | 112 | 0.71 | E | 127 | 0.69 |
| 44 | L | 63 | 0.35 | I | 38 | 0.21 |
| 45 | L | 28 | 0.16 | L | 23 | 0.13 |
| 46 | A | 53 | 0.47 | A | 59 | 0.52 |
| 47 | E | 116 | 0.63 | E | 98 | 0.54 |
| 48 | A | 0 | 0.00 | A | 0 | 0.00 |
| 49 | K | 68 | 0.32 | K | 73 | 0.35 |
| 50 | K | 90 | 0.43 | K | 70 | 0.33 |
| 51 | L | 56 | 0.31 | L | 59 | 0.33 |
| 52 | N | 3 | 0.02 | N | 4 | 0.03 |
| 53 | D | 13 | 0.09 | D | 14 | 0.09 |
| 54 | A | 62 | 0.55 | A | 60 | 0.53 |
| 55 | Q | 87 | 0.46 | Q | 90 | 0.48 |
| 56 | A | 52 | 0.46 | A | 29 | 0.26 |
| 57 | P | 88 | 0.62 | P | 112 | 0.78 |
| 58 | K | 178 | 0.84 | K | 188 | 0.89 |

The amino acid residues with rASA values smaller than the value of Gly-29 (in this case, 0.44), in other words, the amino acid residues with lower solvent-accessible areas, were excluded from candidates to be substituted by acidic amino acids. Also, amino acid residues associated with the specific interaction with IgG, residues located at the domain ends, Pro residue important to keep the main chain structure, wild-type acidic amino acid residues, and other amino acids were excluded to select amino acid residue candidates to be substituted by acidic amino acids. As a result, Lys-7, His-18, Ser-39, Val-40, Ala-46, Ala-54, and Gln-55 were selected.

Next, the mutation effect was verified on the selected seven amino acid residues. The ligand model used to verify the effect was the C domain in the present example. The C domain was selected because the C domain contains a larger amount of acidic amino acids than other domains; for example, the C domain has the acidic amino acid Glu at position 43, at which other domains have Asn. A C domain variant C-G29A/S33E.1d (SEQ ID NO:3) was used as the amino acid sequence before introduction of mutations, which was prepared by introducing two mutations (the G29A mutation for improving the chemical stability of the ligand and the S33E mutation for eliminating binding to the VH3-type Fab region) into the amino acid sequence of the C domain.

A three-dimensional structure model of C-G29A/S33E.1d was prepared using the three-dimensional structure of the Z domain as a template by the protein 3D structure homology modeling software: Homology Modeling for HyperChem (Institute of Molecular Function). In the preparation, optimization of side-chain rotamers and energy minimization using molecular force field potentials were performed by the standard functions provided with the software. The rASA values of this three-dimensional structure model are also shown in Table 1.

Although some residues show rASA values that are greatly different from those in the Z domain, the mutation sites in the present example were selected based on the purely empirically determined three-dimensional structure of the Z domain.

Subsequently, three-dimensional structure models of variants were prepared by substituting each of the selected seven amino acid residues with aspartic acid or glutamic acid. Then, the ASA (ASA-Fc) of the residues (Phe-5, Gln-9, Gln-10, Asn-11, Phe-13, Tyr-14, Leu-17, Asn-28, Ile-31, Gln-32, Lys-35) that specifically interact with the Fc region of IgG was calculated.

Then, the correlation coefficient (Corr.-Fc) of the ASA-Fc of the three-dimensional structure model of each variant relative to the ASA-Fc of the three-dimensional structure model of C-G29A/S33E.1d was calculated. The results are shown in Table 2.

**[Table 2]**

| | Correlation coefficient (Corr.-Fc) | | |
|---|---|---|---|
| Mutation residues | Asp mutation | Glu mutation | Average |
| Lys-07 | 0.96 | 0.95 | 0.96 |
| His-18 | 0.96 | 0.97 | 0.97 |
| Ser-39 | 0.95 | 0.89 | 0.92 |
| Val-40 | 0.97 | 0.94 | 0.96 |
| Ala-46 | 0.94 | 0.96 | 0.95 |
| Ala-54 | 0.91 | 0.92 | 0.92 |
| Gin-55 | 0.98 | 0.98 | 0.98 |

The residues Ser-39 and Ala-54 each of which gave an average Corr.-Fc of lower than 0.95 when substituted by aspartic acid or glutamic acid were considered to have a higher risk of changing the specific IgG binding by mutations than the other residues.

Next, three-dimensional models of all multiple-residue variants (3 to the power of 5 = 243 variants) in which each of the five amino acid residues Lys-7, His-18, Val-40, Ala-46, and Gln-55, excluding Ser-39 and Ala-54, was one of the three amino acids: wild-type, aspartic acid, and glutamic acid, were prepared. The Corr.-Fc of the models was calculated.

Also, the root mean square deviation (RMSD; indicated as RMSD-Fc in this case where the residues are limited) from the three-dimensional structure model of C-G29A/S33E.1d was calculated for all atoms in each of the residues that specifically interact with the Fc region of IgG described above. The Corr.-Fc and RMSD-Fc values of the multiple-residue variants are shown in Tables 3 and 4.

**[Table 3]**

| Mutations | Corr.-Fc | RMSD-Fc | | Mutations | Corr.-Fc | RMSD-Fc |
|---|---|---|---|---|---|---|
| [Charge: -4] | | | | K7K H18E V40E A46D Q55Q | 0.952 | 0.902 |
| K7K H18H V40V A46A Q55D | 0.981 | 0.668 | | K7K H18E V40D A46A Q55E | 0.960 | 0.968 |
| K7K H18H V40V A46A Q55E | 0.975 | 0.819 | | K7D H18E V40V A46A Q66Q | 0.950 | 0.855 |
| K7K H18H V40D A46A Q55Q | 0.969 | 1.096 | | K7D H18H V40V A46E Q65Q | 0.949 | 1.177 |
| K7K H18E V40V A46A Q55Q | 0.966 | 0.798 | | K7K H18E V40V A46E Q55E | 0.949 | 0.916 |
| K7K H18D V40V A46A Q55Q | 0.960 | 0.903 | | K7D H18H V40D A46A Q55Q | 0.946 | 0.768 |
| K7K H18H V40V A46E Q55Q | 0.957 | 0.864 | | K7D H18H V40V A46A Q55D | 0.943 | 0.851 |
| K7K H18H V40V A46D Q55Q | 0.946 | 0.999 | | K7K H18D V40V A46E Q65D | 0.943 | 1.033 |
| K7K H18H V40E A46A Q55Q | 0.940 | 0.976 | | K7K H18H V40E A46D Q55E | 0.943 | 0.881 |
| [Charge: -5] | | | | K7K H18E V40D A46E Q55Q | 0.941 | 0.911 |
| K7K H18H V40D A46D Q55Q | 0.981 | 0.788 | | K7K H18E V40E A46A Q55E | 0.941 | 0.787 |
| K7K H18H V40D A46A Q55E | 0.977 | 1.044 | | K7E H18H V40D A46A Q55Q | 0.941 | 0.755 |
| K7K H18H V40V A46D Q55D | 0.975 | 0.792 | | K7K H18D V40D A46E Q55Q | 0.938 | 1.051 |
| K7K H18H V40V A46D Q55E | 0.975 | 0.886 | | K7E H18D V40V A46A Q55Q | 0.938 | 1.253 |
| K7K H18H V40E A46A Q55E | 0.976 | 0.902 | | K7K H18E V40D A46A Q55D | 0.937 | 0.978 |
| K7K H18H V40E A46E Q55Q | 0.973 | 0.932 | | K7K H18D V40E A46A Q55E | 0.925 | 1.002 |
| K7K H18H V40E A46D Q55Q | 0.971 | 0.801 | | K7K H18H V40D A46E Q55E | 0.922 | 0.950 |
| K7K H18D V40D A46A Q55Q | 0.966 | 0.804 | | K7D H18H V40E A46A Q55Q | 0.914 | 1.104 |
| K7K H18D V40V A46D Q55Q | 0.963 | 1.000 | | K7E H18H V40V A46A Q55D | 0.912 | 0.959 |
| K7K H18H V40E A46A Q55D | 0.963 | 0.805 | | K7K H18D V40D A46D Q55Q | 0.905 | 1.029 |
| K7D H18H V40V A46A Q55Q | 0.960 | 1.010 | | K7K H18H V40E A46E Q55E | 0.899 | 1.044 |
| K7K H18E V40D A46A Q55Q | 0.959 | 0.872 | | K7K H18E V40V A46D Q55E | 0.896 | 1.120 |
| K7K H18H V40V A46E Q55D | 0.956 | 0.853 | | K7E H18H V40E A46A Q55Q | 0.894 | 0.945 |
| K7K H18E V40V A46A Q55E | 0.956 | 0.895 | | [Charge: -7] | | |
| K7K H18E V40V A46E Q55Q | 0.955 | 0.982 | | K7D H18H V40D A46E Q55Q | 0.991 | 0.832 |
| K7K H18H V40D A46A Q55D | 0.953 | 1.036 | | K7E H18H V40D A46D Q55Q | 0.989 | 0.882 |
| K7K H18E V40V A46D Q55Q | 0.953 | 1.002 | | K7K H18E V40D A46E Q55E | 0.987 | 0.913 |
| K7K H18D V40V A46A Q55E | 0.948 | 1.170 | | K7E H18E V40V A46A Q55E | 0.983 | 0.588 |
| K7E H18H V40V A46A Q55Q | 0.948 | 0.984 | | K7D H18H V40D A46D Q55Q | 0.982 | 0.727 |
| K7K H18H V40D A46E Q55Q | 0.943 | 0.824 | | K7E H18D V40D A46A Q55Q | 0.982 | 0.879 |
| K7K H18E V40E A46A Q55Q | 0.939 | 1.048 | | K7D H18E V40V A46A Q55E | 0.981 | 0.946 |
| K7K H18D V40V A46A Q55D | 0.923 | 1.062 | | K7K H18D V40D A46E Q55E | 0.978 | 0.831 |
| K7K H18D V40V A46E Q55Q | 0.917 | 1.178 | | K7D H18E V40V A46E Q55Q | 0.977 | 1.070 |
| K7K H18E V40V A46A Q55D | 0.916 | 1.067 | | K7E H18E V40V A46D Q55Q | 0.977 | 0.632 |
| K7K H18D V40E A46A Q55Q | 0.910 | 1.138 | | K7D H18D V40D A46A Q55Q | 0.976 | 0.921 |
| K7K H18H V40V A46E Q55E | 0.870 | 0.861 | | K7K H18E V40D A46D Q55E | 0.975 | 0.676 |
| [charge: -6] | | | | K7D H18D V40V A46E Q55Q | 0.975 | 1.079 |
| K7K H18D V40V A46D Q55D | 0.990 | 0.923 | | K7E H18H V40D A46E Q55Q | 0.974 | 1.138 |
| K7D H18D V40V A46A Q55Q | 0.986 | 0.963 | | K7E H18H V40V A46E Q65D | 0.974 | 0.695 |
| K7K H18H V40D A46E Q55D | 0.985 | 0.804 | | K7E H18D V40V A46A Q55D | 0.970 | 0.856 |
| K7K H18H V40E A46D Q55D | 0.982 | 0.955 | | K7E H18D V40V A46D Q55Q | 0.970 | 1.116 |
| K7E H18H V40V A48E Q55Q | 0.981 | 0.952 | | K7E H18H V40E A46D Q55Q | 0.970 | 0.967 |
| K7K H18E V40V A46D Q55D | 0.979 | 0.924 | | K7D H18H V40E A46D Q55Q | 0.969 | 0,688 |
| K7E H18H V40V A46A Q55E | 0.979 | 1.089 | | K7E H18H V40E A48A Q55D | 0.968 | 0.763 |
| K7K H18H V40D A46D Q55E | 0.978 | 1.074 | | K7E H18E V40D A46A Q55Q | 0.968 | 0.976 |
| K7K H18E V40V A46E Q55D | 0.977 | 0.945 | | K7E H18H V40E A46A Q55E | 0.968 | 0.986 |
| K7D H18H V40V A46D Q55Q | 0.977 | 0.916 | | K7E H18E V40V A46A Q55D | 0.967 | 0.873 |
| K7K H18H V40E A46E Q55D | 0.977 | 0.890 | | K7D H18H V40D A46A Q65E | 0.966 | 1.009 |
| K7E H18H V40V A46D Q55Q | 0.977 | 1.026 | | K7D H18E V40V A46A Q55D | 0.966 | 0.784 |
| K7K H18E V40D A46D Q55Q | 0.976 | 0.988 | | K7E H18E V40V A46E Q55Q | 0.964 | 1.061 |
| K7D H18H V40V A46A Q55E | 0.976 | 0.921 | | K7K H18D V40D A46D Q55D | 0.963 | 1.137 |
| K7K H18E V40E A46A Q55D | 0.974 | 0.727 | | K7K H18E V40D A46E Q55D | 0,963 | 0.922 |
| K7E H18E V40V A46A Q55Q | 0.973 | 1.147 | | K7E H18H V40D A46A Q66E | 0.962 | 0.843 |
| K7K H18E V40E A46E Q55Q | 0.970 | 1.032 | | K7D H18E V40E A46A Q55Q | 0.960 | 0.931 |
| K7K H18D V40E A46A Q55D | 0.988 | 0.816 | | K7E H18H V40V A46D Q65D | 0.954 | 0.993 |
| K7K H18D V40E A46E Q55Q | 0.966 | 0.919 | | K7D H18E V40V A46D Q55Q | 0,962 | 0.841 |
| K7K H18D V40V A46E Q55E | 0.965 | 1.165 | | K7E H18H V40V A46E Q55E | 0.952 | 1.116 |
| K7K H18D V40D A46A Q55D | 0.964 | 0.860 | | K7K H18E V40E A48D Q55D | 0.951 | 0.903 |
| K7K H18D V40E A46D Q55Q | 0.961 | 1.121 | | K7E H18H V40V A46D Q55E | 0.951 | 0,985 |
| K7K H18D V40V A46D Q55E | 0.960 | 1.008 | | K7K H18E V40E A46D Q55E | 0.950 | 1.041 |
| K7K H18D V40D A46A Q55E | 0.957 | 0.841 | | K7D H18D V40E A46A Q55Q | 0.950 | 1.289 |
| K7K H18H V40D A46D Q55D | 0.954 | 1.155 | | K7K H18D V40D A46E Q66D | 0.949 | 1.194 |

**[Table 4]**

| Mutations | Corr.-Fc | RMSD-Fc | | Mutations | Carr.-Fc | RMSD-Fc |
|---|---|---|---|---|---|---|
| K7D H18H V40E A46A Q55E | 0.949 | 1.344 | | K7D H18D V40D A46A Q55D | 0.954 | 0.943 |
| K7D H18H V40E A46A Q55D | 0.947 | 0.792 | | K7D H18D V40D A46D Q55Q | 0.953 | 1.136 |
| K7K H18E V40E A46E Q66E | 0.947 | 0.889 | | K7E H18E V40V A46D Q55E | 0.952 | 1.204 |
| K7K H18D V40E A46E Q55E | 0.947 | 1.145 | | K7D H18D V40V A46E Q55D | 0.952 | 1.056 |
| K7D H18H V40D A46A Q55D | 0.946 | 0.937 | | K7D H18E V40E A46A Q55E | 0.950 | 1,063 |
| K7E H18D V40V A46E Q55Q | 0.944 | 1.207 | | K7D H18E V40D A46D Q55Q | 0.948 | 0.956 |
| K7K H18E V40D A46D Q55D | 0.943 | 0.852 | | K7D H18D V40E A46A Q66E | 0.946 | 1.032 |
| K7E H18D V40E A46A Q55Q | 0.943 | 0.776 | | K7D H18D V40E A46D Q55Q | 0.946 | 1.249 |
| K7E H18E V40E A46A Q55Q | 0.943 | 0.888 | | K7D H18E V40V A46D Q55E | 0.945 | 0.855 |
| K7E H18H V40E A46E Q55Q | 0.942 | 1.059 | | K7D H18H V40E A46D Q55E | 0.945 | 0.994 |
| K7D H18E V40D A46A Q55Q | 0.939 | 0.880 | | K7E H18D V40V A46D Q55E | 0.945 | 0.860 |
| K7D H18D V40V A46D Q55Q | 0.938 | 0,816 | | K7D H18E V40D A46E Q55Q | 0.942 | 1.116 |
| K7E H18D V40V A46A Q55E | 0.938 | 1.064 | | K7E H18E V40D A46D Q55Q | 0.941 | 0.993 |
| K7D H18H V40V A46D Q55E | 0.937 | 1.118 | | K7D H18H V40D A46D Q55D | 0.941 | 1.022 |
| K7K H18D V40E A46D Q55E | 0.935 | 0.842 | | K7E H18H V40D A46D Q55D | 0.936 | 0.900 |
| K7K H18D V40E A46E Q55D | 0.934 | 1.076 | | K7D H18D V40E A48A Q65D | 0.934 | 0.971 |
| K7D H18D V40V A46A Q55D | 0.934 | 1.276 | | K7E H18D V40E A46E Q55Q | 0.931 | 1.044 |
| K7D H18H V40E A46E Q55Q | 0.934 | 0.904 | | K7D H18E V40E A46D Q55Q | 0.926 | 0.969 |
| K7K H18E V40E A46E Q55D | 0.927 | 1.021 | | K7E H18H V40D A46D Q55E | 0.926 | 1.011 |
| K7D H18H V40V A46E Q55D | 0.923 | 1.173 | | K7D H18E V40V A46D Q55D | 0.920 | 1.001 |
| K7D H18H V40V A46D Q55D | 0.914 | 0.933 | | K7E H18D V40E A46D Q55Q | 0.916 | 1.148 |
| K7E H18H V40D A46A Q55D | 0.912 | 0.818 | | K7D H18E V40D A46A Q55D | 0.910 | 0.937 |
| K7K H18D V40E A46D Q55D | 0.910 | 0.963 | | K7D H18D V40V A46D Q55E | 0.907 | 0.860 |
| K7K H18D V40D A46D Q55E | 0.905 | 1.203 | | K7E H18H V40D A46E Q55E | 0.906 | 1.160 |
| K7D H18H V40V A46E Q55E | 0.902 | 1.023 | | K7E H18H V40E A46D Q55E | 0.892 | 1.016 |
| K7D H18D V40V A46A Q55E | 0.853 | 1.162 | | K7D H18D V40D A46A Q55E | 0.889 | 0.962 |
| [charge: -8] | | | | K7E H18E V40E A46A Q55E | 0.884 | 1.033 |
| K7E H18D V40E A46A Q55D | 0.992 | 1.044 | | K7E H18H V40D A46E Q55D | 0.883 | 1.031 |
| K7D H18H V40E A46D Q55D | 0.992 | 0.916 | | K7E H18D V40E A46A Q55E | 0.879 | 1.171 |
| K7E H18E V40D A46A Q55E | 0.991 | 0.901 | | [Charge: -9] | | |
| K7E H18E V40V A46E Q55E | 0.986 | 0.787 | | K7E H18D V40D A460 Q55E | 0.989 | 0.719 |
| K7E H18D V40V A46E Q55D | 0.985 | 1.097 | | K7E H18D V40D A46D Q55D | 0.987 | 0.818 |
| K7D H18D V40V A46E Q55E | 0.982 | 0.729 | | K7E H18D V40E A48E Q55E | 0.985 | 1.086 |
| K7D H18D V40V A46D Q55D | 0.981 | 0.900 | | K7D H18D V40E A46E Q55E | 0.982 | 0.895 |
| K7E H18E V40E A46A Q55D | 0.981 | 0.987 | | K7D H18E V40E A46D Q55D | 0.980 | 0.778 |
| K7D H18E V40V A46E Q55E | 0.980 | 1.222 | | K7D H18E V40E A46D Q55E | 0.980 | 0.761 |
| K7E H18D V40V A46E Q55E | 0.980 | 0.885 | | K7E H18E V40E A46E Q55E | 0.977 | 0.978 |
| K7E H18E V40E A48D Q55Q | 0.977 | 0.943 | | K7D H18D V40D A46D Q55D | 0.976 | 1.020 |
| K7D H18D V40E A46E Q55Q | 0.977 | 0.942 | | K7D H18E V40E A48E Q55E | 0.976 | 0.870 |
| K7D H18E V40E A46A Q55D | 0.976 | 0.927 | | K7E H18E V40E A46E Q55D | 0.974 | 0.994 |
| K7E H18D V40D A48A Q65E | 0.976 | 0.927 | | K7E H18D V40D A46E Q55E | 0.970 | 0.695 |
| K7D H18H V40D A46E Q55E | 0.974 | 0,857 | | K7D H18E V40D A46D Q55D | 0.964 | 0.852 |
| K7D H18E V40D A46A Q65E | 0.973 | 0.796 | | K7D H18D V40E A46E Q66D | 0.962 | 0.962 |
| K7E H18E V40V A46E Q55D | 0.973 | 0.635 | | K7E H18E V40D A46D Q66D | 0.959 | 0.956 |
| K7E H18D V40D A46A Q55D | 0.971 | 0.782 | | K7E H18D V40E A46D Q55D | 0.959 | 0.906 |
| K7E H18E V40V A46D Q55D | 0.971 | 0.713 | | K7D H18E V40D A46E Q66E | 0.958 | 0.897 |
| K7E H18H V40E A46E Q55E | 0.971 | 0.877 | | K7D H18D V40D A46E Q55E | 0.957 | 1.089 |
| K7E H18E V40D A46A Q55D | 0.970 | 0.939 | | K7E H18E V40D A46E Q55E | 0.957 | 0.659 |
| K7D H18H V40D A46E Q55D | 0.969 | 0.914 | | K7E H18E V40E A46D Q55D | 0.955 | 1.022 |
| K7E H18H V40E A46D Q55D | 0.969 | 0.747 | | K7D H18D V40D A46E Q55D | 0.955 | 0.936 |
| K7D H18H V40E A46E Q55E | 0.969 | 0.933 | | K7D H18D V40E A46D Q55D | 0.950 | 0.817 |
| K7D H18H V40D A46D Q55E | 0.965 | 0.904 | | K7E H18E V40D A46D Q55E | 0.943 | 0.963 |
| K7D H18H V40E A46E Q55D | 0.962 | 0.926 | | K7D H18E V40D A46D Q55E | 0.936 | 1.070 |
| K7E H18D V40D A46D Q55Q | 0.962 | 0.809 | | K7E H18E V40E A46D Q55E | 0.934 | 1.250 |
| K7E H18D V40V A46D Q55D | 0.960 | 0.771 | | K7E H18D V40D A46E Q55D | 0.932 | 1.120 |
| K7E H18E V40D A46E Q55Q | 0.959 | 0.962 | | K7E H18D V40E A46D Q55E | 0.931 | 0.846 |
| K7E H18D V40D A46E Q55Q | 0.958 | 0.995 | | K7D H18E V40E A46E Q55D | 0.930 | 0.780 |
| K7D H18D V40D A46E Q55Q | 0.957 | 0.718 | | K7D H18D V40E A46D Q66E | 0.924 | 1.064 |
| K7D H18E V40V A46E Q55D | 0.956 | 1.039 | | K7E H18D V40E A46E Q55D | 0.920 | 0.964 |
| K7E H18H V40E A46E Q55D | 0.955 | 0.881 | | K7E H18E V40D A46E Q55D | 0.914 | 0.852 |
| K7D H18E V40E A46E Q55Q | 0.954 | 0.910 | | K7D H18E V40D A46E Q55D | 0.907 | 1.085 |
| K7E H18E V40E A46E Q55Q | 0.954 | 0.866 | | K7D H18D V40D A46D Q55E | 0.902 | 1.610 |

Since variants with high Corr.-Fc values are considered to have a low risk of changing the specific IgG binding, such variants are preferred as ligands used in the present invention. From the same standpoint, variants with low RMSD-Fc values are also preferred as ligands used in the present invention.

Based on the three-dimensional structure models, the surface charge of the variants was also estimated by software for estimation of molecular surface charge, Adaptive Poisson-Boltzmann Solver (APBS, http://www.poissonboltzmann.org/apbs). The results showed that introduction of acidic amino acid residues at proper positions caused only small changes in charge on the surfaces interacting with IgG, but increased negatively charged areas on the other surfaces.

### (Example 2) Acquisition of C domain variants of Protein A with acidic amino acids introduced therein

Peptides obtained by introducing the substitutions with acidic amino acids designed in Example 1 into the C domain of Protein A were acquired using an *E. coli* expression system.

The following variants (1) to (3) were prepared by introducing the substitutions with acidic amino acids designed in Example 1 into C-G29A/S33E.1d. As a comparative example, (0) C-G29A/S33E.1d was used. To distinguish the substitutions with acidic amino acids in the present invention from the mutations (G29A/S33E) of the comparative example, the substitutions in the present invention are all indicated after G29A/S33E.
(0) C-G29A/S33E.1d (SEQ ID NO:3, comparative example)
(1) C-G29A/S33E/Q55E.1d (SEQ ID NO:4)
(2) C-G29A/S33E/K7E/A46E.1d (SEQ ID NO:5)
(3) C-G29A/S33E/K7E/H18D/V40D/A46D/Q55E.1d (SEQ ID NO:6)

The DNA encoding each variant was inserted into a commercially available *E. coli* expression vector, pGEX6P-1 (GE Healthcare Bio-Sciences) which can express and purify a single-domain protein fused with GST (glutathione S-transferase) for standardization of peptide purification procedures to construct an expression vector. Using the expression vector, the expression system *E. coli* was transformed. The process is summarized below.

The method for constructing an expression vector for preparing (0) C-G29A/S33E.1d (comparative example) is described as an example. The base sequence (SEQ ID NO:7) encoding the peptide was designed by reverse translation of the amino acid sequence of C-G29A/S33E.1d (SEQ ID NO:3).

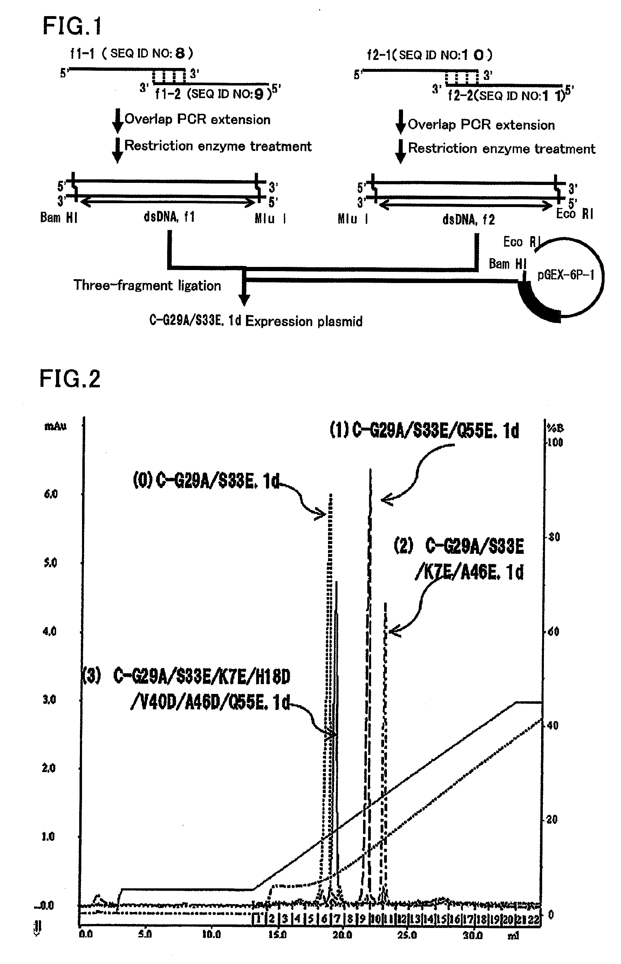

The DNA encoding C-G29A/S33E.1d was prepared by ligating two double-stranded DNA fragments (f1 and f2) cut with restriction enzymes, and was inserted into the multicloning site of the expression vector. Actually, preparation of the encoding DNA and insertion into the vector were simultaneously performed by a three-fragment ligation in which the three double-stranded DNA fragments: the two double-stranded DNA fragments and the expression vector were mixed and ligated. The two double-stranded DNA fragments were prepared by extending two single-stranded oligo DNA fragments (f1-1/f1-2 or f2-1/f2-2) containing a complementary region of about ten bases with each other by overlap PCR to prepare the desired double-stranded DNA fragment. The method for constructing an expression vector is summarized in Fig. 1.

Specifically, the expression vector was constructed by the following procedures. Single-stranded oligo DNA fragments, f1-1 (SEQ ID NO:8) and f1-2 (SEQ ID NO:9) were synthesized by an external institution (Sigma-Genosys). These fragments were subjected to an overlap PCR reaction using Blend Taq (Toyobo Co., Ltd.) as a polymerase. The PCR reaction product was subjected to agarose electrophoresis and a double-stranded DNA extracted by cutting out the target band was cut with the restriction enzymes BamHI and MluI (both from Takara Bio, Inc.). Similarly, single-stranded oligo DNA fragments, f2-1 (SEQ ID NO:10) and f2-2 (SEQ ID NO:11) were synthesized by the external institution and subjected to an overlap PCR reaction to synthesize and extract a double-stranded DNA, which was cut with the restriction enzymes MluI and EcoRI (both from Takara Bio, Inc.). Next, these two double-stranded DNA fragments were subcloned at the BamHI/EcoRI site in the multicloning site of the plasmid vector pGEX-6P-1. The ligation reaction in the subcloning was carried out using Ligation High (Toyobo Co., Ltd.) in accordance with the protocol attached to the product.

Using the plasmid vector pGEX-6P-1 containing the DNA encoding the peptide, an *E. coli* competent cell, HB101 (Takara Bio, Inc.) was transformed according to the attached protocol. The plasmid vector pGEX-6P-1 allows us to produce C-G29A/S33E.1d fused with GST.

Using a plasmid purification kit ("Wizard Plus SV Minipreps DNA Purification System" from Promega Corporation), the expression vector was extracted from the transformant in accordance with the standard protocol attached to the kit.

The base sequence of the encoding DNA in the expression vector was determined using a DNA sequencer ("3130x1 Genetic Analyzer" from Applied Biosystems). Using a gene analysis kit ("BigDye Terminator v.1.1 Cycle Sequencing Kit" from Applied Biosystems) and a DNA primer (GE Healthcare Bio-Sciences) for sequencing the plasmid vector pGEX-6P-1, a sequencing PCR reaction was performed according to the attached protocol. The reaction product was purified in accordance with the protocol attached to the plasmid purification kit BigDye XTerminator Purification Kit (Applied Biosystems), and used in base sequence analysis.

For the DNAs encoding the above C domain variants (1) to (3), the base sequences encoding the peptides were designed by reverse translation of the amino acid sequences, and expression vectors and transformants containing the encoding DNAs were prepared by the same methods as described above. Combinations of the SEQ ID NOs of the DNA sequences encoding the C domain variants (1) to (3) and the single-stranded oligo DNA fragments (f1-1, f1-2, f2-1, f2-2) used in the synthesis thereof are shown in Table 5. The DNAs encoding the peptides of the present invention can all be synthesized by external institutions (for example, Eurogentec) if they each have a length of about 200 bases (which can encode a protein of about 60 residues).

**[Table 5]**

| Variant | Amino acid | Encoding DNA | Single-stranded oligo DNA | | | |
|---|---|---|---|---|---|---|
| | | | f1-1 | f1-2 | f2-1 | f2-2 |
| C-G29A/S33E.1d | 3 | 7 | 8 | 9 | 10 | 11 |
| C-G29A/S33E/Q55E.1d | 4 | 12 | 8 | 9 | 10 | 13 |
| C-G29A/S33E/K7E/A46E.1d | 5 | 14 | 15 | 9 | 10 | 16 |
| C-G29A/S33E/K7E/H18D/V40D/A46D/Q55E.1d | 6 | 17 | 15 | 18 | 19 | 20 |

Each transformant was cultured in 2xYT medium containing ampicillin at 37°C overnight. The culture fluid was inoculated in an about 100-fold volume of 2xYT medium containing ampicillin, and cultured at 30°C for about three hours. After that, IPTG (isopropyl-1-thio-β-D-galactoside) was added to a final concentration of 0.2 mM, followed by culture at 30°C for 24 more hours.

After the completion of culture, cells were collected by centrifugation and resuspended in 5 mL of PBS buffer (for about each 0.75 L of the medium). The cells were disrupted by sonication and centrifuged to separate a supernatant fraction (cell-free extract) and an insoluble fraction. When the gene of interest is introduced into the multicloning site of pGEX-6P-1 vector, it is expressed as a fusion protein with GST attached at the N terminal. The supernatant fraction and the insoluble fraction were each analyzed by SDS electrophoresis. A peptide band at a position corresponding to a molecular weight of about 33,000, which was presumed to be induced by IPTG, was observed for all the supernatant fractions (cell-free extracts) prepared from the transformant cultures.

The GST fusion peptides were partially purified from the cell-free extracts containing the GST fusion peptides by affinity chromatography using a GSTrap HP column (GE Healthcare Bio-Sciences), which has an affinity for GST. Specifically, each cell-free extract was applied to the GSTrap HP column, and the column was washed with a standard buffer (20 mM NaH₂PO₄-Na₂HPO₄, 150 mM NaCl, pH 7.4). Then, the GST fusion peptide of interest was eluted with an elution buffer (50 mM Tris-HCl, 20 mM glutathione, pH 8.0).

When the gene is introduced into the multicloning site of pGEX-6P-1 vector, an amino acid sequence that sequence-specific protease PreScission Protease (GE Healthcare Bio-Sciences) recognizes to cleave GST off is introduced between GST and the protein of interest. A GST cleavage reaction was performed using PreScission Protease according to the attached protocol.

The GST-cleaved samples were subjected to gel filtration chromatography using a Superdex 75 10/300 GL column (GE Healthcare Bio-Sciences) to purify the peptides of interest. Each reaction solution was applied to the Superdex 75 10/300 GL column equilibrated with the standard buffer to separate and purify the protein of interest from the cleaved GST and PreScission Protease. All the above peptide purification procedures by chromatography using the column were carried out in an AKTAprime plus system (GE Healthcare Bio-Sciences). The amino acid sequence of each GST-cleaved peptide contains Gly-Pro-Leu-Gly-Ser derived from the vector pGEX-6P-1 on the N-terminal side.

### (Example 3) Analysis for affinity of acquired C domain variants for human IgG

The peptides obtained in Example 2 were analyzed for affinity for an immunoglobulin with a surface plasmon resonance-based biosensor, Biacore 3000 (GE Healthcare Bio-Sciences). A human immunoglobulin G drug (hereinafter, referred to as human IgG) separated from human plasma was immobilized on a sensor chip, and each peptide was flowed on the chip to detect an interaction between them.

The immobilization of the human IgG on the sensor chip CM5 was carried out by amine coupling using N-hydroxysuccinimide (NHS) and N-ethyl-N'-(3-dimethylaminopropyl)carbodiimide hydrochloride (EDC), and ethanolamine was used for blocking (the sensor chip and the immobilization reagents are all available from GE Healthcare Bio-Sciences). The human IgG solution was prepared by dissolving γ-globulin (Nihon Pharmaceutical Co., Ltd.) in a standard buffer (20 mM NaH₂PO₄-Na₂HPO₄, 150 mM NaCl, pH 7.4) to a concentration of 1.0 mg/mL. The human IgG solution was diluted to 1/10 in an immobilization buffer (10 mM CH₃COOH-CH₃COONa, pH 4.5), and then immobilized on the sensor chip in accordance with the protocol attached to the Biacore 3000 (about 3000 RU (resonance unit)). A reference cell used as a negative control was also prepared by immobilizing ethanolamine on another flow cell on the chip after activation by EDC/NHS.

The peptides were appropriately prepared at concentrations ranging from 10 to 1000 nM in a running buffer (20 mM NaH₂PO₄-Na₂HPO₄, 150 mM NaCl, 0.005% P-20, pH 7.4) (three solutions of different concentrations were prepared for each peptide), and each solution was injected to the sensor chip at a flow rate of 40 µL/min for 60 seconds. Binding response sensorgrams measured at 25°C were sequentially recorded during injection (binding phase, 60 seconds) and after injection (dissociation phase, 60 seconds). After each recording, the sensor chip was regenerated with 25 mM NaOH (for 30 seconds). This operation was performed to remove the peptide remaining on the sensor chip, and was confirmed that the binding activity of the immobilized human IgG was almost completely restored. The affinity constant of the peptides for human IgG (K_{A}=kₒₙ/k_{off}) was calculated by performing a fitting analysis on the obtained binding response sensorgrams (the binding response sensorgrams obtained by subtracting the binding response sensorgram of the reference cell) using a 1:1 binding model by the BIA evaluation software attached to the system. The results are shown in Table 6.

The single domain-type C domain variants showed binding activity to human IgG. The binding parameters of the variant (1) C-G29A/S33E/Q55E.1d and the variant (2) C-G29A/S33E/K7E/A46E.1d were similar to those of (0) C-G29A/S33E.1d (comparative example). As for the binding parameters of the variant (3) C-G29A/S33E/K7E/H18D/V40D/A46D/Q55E.1d, the dissociation rate constant k_{off} was similar to that of C-G29A/S33E.1d, but the binding rate constant kₒₙ was significantly smaller therethan and the affinity constant K_{A} was about 1/10 thereof. However, as a moderate decrease in binding strength can be advantageous in separating (eluting) the antibody held, the minimum binding strength required was maintained.

**[Table 6]**

| Variant | kon [x10⁶ M⁻¹s] | k_{off} [x10⁻³ s⁻¹] | Kₐ [x10⁷ M⁻¹] |
|---|---|---|---|
| (0) C-G29A/S33E.1d | 3.21 | 5.85 | 5.48 |
| (1) C-G29A/S33E/Q55E.1d | 3.58 | 5.82 | 6.15 |
| (2) C-G29A/S33E/K7E/A46E.1d | 2.76 | 7.09 | 3.90 |
| (3) C-G29A/S33E/K7E/H18D/V40D/A46D/Q55E.1d | 0.29 | 5.19 | 0.57 |

### (Example 4) Analysis for molecular surface properties of acquired C domain variants

The single domain-type C domain variants acquired in Example 2 were subjected to anion exchange chromatography to analyze the difference in anionic properties between the variants.

Protein solutions of the single domain-type C domain variants were concentrated while being diluted to about 1/100 in an anion exchange buffer A (50 mM Tris-HCl, pH 8.0) using a centrifugal filter unit (Amicon from Merck Millipore Corporation). Using the AKTAprime plus system, these samples were applied to an anion exchange column, Mono Q column (GE Healthcare Bio-Sciences) equilibrated with the anion exchange buffer A, and then washed with the anion exchange buffer A. Thereafter, the proteins of interest were eluted and monitored with UV in the process of salt concentration gradient elution (B: 5%→45%, 20CV) using the anion exchange buffer A and an anion exchange buffer B (50 mM Tris-HCl, 1.0 M NaCl, pH 8.0). The overlapped chromatographic profiles are shown in Fig. 2.

As shown in Fig. 2, all the peptides of the present invention had peak retention times longer than (peaks located behind) that of C-G29A/S33E.1d (comparative example). This means that higher salt concentrations are needed to dissociate the peptides of the present invention from the column than that for C-G29A/S33E.1d. The acidic amino acid mutations enhanced the anionic properties of acidic groups (carboxyl groups) on the molecular surface, which verifies the effectiveness of the design in Example 1. The enhancement of the anionic properties of the peptides is considered to increase the cation exchange function when the peptides are immobilized as ligands, thereby improving the antibody separation ability of ligands as compared to those before introduction of mutations. The results of Examples 1 to 4 show that the present invention can effectively alter the surface charge (cation exchange effect) of Protein A without impairing the antibody-binding ability of Protein A.

## Claims

1. An immunoglobulin G-binding peptide, which is any one of the following peptides (1) to (3):
(1) an immunoglobulin G-binding peptide comprising an amino acid sequence comprising a substitution of at least one amino acid selected from the group consisting of amino acids at positions 7, 18, 39, 40, 46, 54, and 55 in the amino acid sequence of SEQ ID NO:1 or the amino acid sequence of SEQ ID NO:2 with an acidic amino acid;
(2) an immunoglobulin G-binding peptide comprising an amino acid sequence further comprising a substitution, insertion, deletion, and/or addition of at least one amino acid at a position other than positions 7, 18, 39, 40, 46, 54, and 55 in the amino acid sequence of the peptide (1); and
(3) an immunoglobulin G-binding peptide comprising an amino acid sequence having at least 80% sequence identity to the amino acid sequence of the peptide (1), wherein the acidic amino acid introduced into the peptide (1) by substitution is retained.

2. The immunoglobulin G-binding peptide according to claim 1,
wherein the acidic amino acid is an acidic amino acid having a carboxyl group in a side chain.

3. The immunoglobulin G-binding peptide according to claim 2,
wherein the acidic amino acid having a carboxyl group in a side chain is aspartic acid or glutamic acid.

4. The immunoglobulin G-binding peptide according to any one of claims 1 to 3,
wherein the amino acid substituted by the acidic amino acid is at least one amino acid selected from the group consisting of amino acids at positions 7, 18, 40, 46, and 55.

5. The immunoglobulin G-binding peptide according to any one of claims 1 to 4,
wherein the amino acid subjected to substitution, insertion, deletion, and/or addition is at least one amino acid selected from the group consisting of amino acids at positions 1, 2, 3, 4, 6, 23, 29, 33, 35, 36, 37, 42, 49, 50, 57, and 58.

6. The immunoglobulin G-binding peptide according to claim 5,
wherein the amino acid subjected to substitution, insertion, deletion, and/or addition is at least one amino acid selected from the group consisting of amino acids at positions 29, 33, 36, and 37.

7. The immunoglobulin G-binding peptide according to any one of claims 1 to 6,
wherein at least two amino acids are substituted by acidic amino acids.

8. The immunoglobulin G-binding peptide according to any one of claims 1 to 7,
wherein at least three amino acids are substituted by acidic amino acids.

9. The immunoglobulin G-binding peptide according to any one of claims 1 to 8,
wherein at least four amino acids are substituted by acidic amino acids.

10. An immunoglobulin G-binding peptide, comprising a plurality of domains in which at least two of the immunoglobulin G-binding peptides according to any of claims 1 to 9 are connected.

11. A DNA, comprising a base sequence encoding the immunoglobulin G-binding peptide according to any one of claims 1 to 10.

12. A vector, comprising the DNA according to claim 11.

13. A transformant, obtained by introducing the vector according to claim 12 into a host cell.

14. An affinity separation matrix, comprising the immunoglobulin G-binding peptide according to any one of claims 1 to 10 immobilized on a water-insoluble carrier.

15. A method for producing immunoglobulin G or an immunoglobulin G derivative, comprising
bringing immunoglobulin G or an immunoglobulin G derivative into contact with the affinity separation matrix according to claim 14, and
separating the immunoglobulin G or immunoglobulin G derivative bound to the affinity separation matrix from the affinity separation matrix.

16. A method for producing a modified ligand for affinity purification, comprising
increasing the number of acidic amino acids in an amino acid sequence of a ligand for affinity purification to improve a cation exchange ability of the ligand.

17. The method for producing a modified ligand for affinity purification according to claim 16,
wherein the number of acidic amino acids is increased by substitution with an acidic amino acid and/or addition of an acidic amino acid.

18. The method for producing a modified ligand for affinity purification according to claim 16 or 17,
wherein the target of affinity purification is immunoglobulin G or an immunoglobulin G derivative.

19. The method for producing a modified ligand for affinity purification according to any one of claims 16 to 18,
wherein the ligand for affinity purification is an immunoglobulin G-binding domain of Protein A, Protein G, or Protein L.

20. The method for producing a modified ligand for affinity purification according to any one of claims 16 to 19,
wherein the number of acidic amino acids is increased by two or more.

21. The method for producing a modified ligand for affinity purification according to any one of claims 16 to 20,
wherein the number of acidic amino acids is increased by three or more.

22. The method for producing a modified ligand for affinity purification according to any one of claims 16 to 21,
wherein the number of acidic amino acids is increased by four or more.


SEQUENCE LISTING

<110>  \212\224\216®\211ï\216Ð\203J\203l\203J KANEKA CORPORATION

<120>
\203A\203t\203B\203j\203e\203B\201[\225ª\227£\203}\203g\203\212\203b\203N\203
X\227p\225ª\227£\224\\213-\211»\203\212\203K\203\223\203h

<130>  B130451WO01

<150>  JP2013-184922

       JP2013-222508

<151>  2013-09-06

       2013-10-25

<160>  20

<170>  PatentIn version 3.5

<210>  1
<211>  58
<212>  PRT
<213>  Staphylococcus aureus

<400>  1

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Gly Phe Ile Gln
            20                  25                  30

Ser Leu Lys Asp Asp Pro Ser Val Ser Lys Glu Ile Leu Ala Glu Ala
        35                  40                  45

Lys Lys Leu Asn Asp Ala Gln Ala Pro Lys
    50                  55

<210>  2
<211>  58
<212>  PRT
<213>  Artificial Sequence

<220>
<223>  SpA mutant

<400>  2

<210>  3
<211>  58
<212>  PRT
<213>  Artificial Sequence

<220>
<223>  SpA mutant

<400>  3

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Glu Leu Lys Asp Asp Pro Ser Val Ser Lys Glu Ile Leu Ala Glu Ala
        35                  40                  45

Lys Lys Leu Asn Asp Ala Gln Ala Pro Lys
    50                  55

<210>  4
<211>  58
<212>  PRT
<213>  Artificial Sequence

<220>
<223>  SpA mutant

<400>  4

Ala Asp Asn Lys Phe Asn Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Glu Leu Lys Asp Asp Pro Ser Val Ser Lys Glu Ile Leu Ala Glu Ala
        35                  40                  45

Lys Lys Leu Asn Asp Ala Glu Ala Pro Lys
    50                  55

<210>  5
<211>  58
<212>  PRT

<213>  Artificial Sequence

<220>
<223>  SpA mutant

<400>  5

Ala Asp Asn Lys Phe Asn Glu Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Glu Leu Lys Asp Asp Pro Ser Val Ser Lys Glu Ile Leu Glu Glu Ala
        35                  40                  45

Lys Lys Leu Asn Asp Ala Gln Ala Pro Lys
    50                  55

<210>  6
<211>  58
<212>  PRT
<213>  Artificial Sequence

<220>
<223>  SpA mutant

<400>  6

Ala Asp Asn Lys Phe Asn Glu Glu Gln Gln Asn Ala Phe Tyr Glu Ile
1               5                   10                  15

Leu Asp Leu Pro Asn Leu Thr Glu Glu Gln Arg Asn Ala Phe Ile Gln
            20                  25                  30

Glu Leu Lys Asp Asp Pro Ser Asp Ser Lys Glu Ile Leu Asp Glu Ala
        35                  40                  45

Lys Lys Leu Asn Asp Ala Glu Ala Pro Lys
    50                  55

<210>  7
<211>  174
<212>  DNA
<213>  Artificial Sequence

<220>
<223>  Code DNA

<400>  7
gctgacaaca agtttaacaa agaacaacaa aatgctttct atgagatcct gcatttacct      60

aacttaactg aggaacaacg taacgcgttc attcaagagc ttaaggacga tccttcagtg      120

agcaaagaga tcctggcaga agctaaaaag cttaacgatg ctcaggcgcc aaaa      174

<210>  8
<211>  60
<212>  DNA
<213>  Artificial Sequence

<220>
<223>  synthetic oligonucleotide

<400>  8
cgtggatccg ctgacaacaa gtttaacaaa gaacaacaaa atgctttcta tgagatcctg        60


<210>  9
<211>  51
<212>  DNA
<213>  Artificial Sequence

<220>
<223>  synthetic oligonucleotide

<400>  9
aatgaacgcg ttacgttgtt cctcagttaa gttaggtaaa tgcaggatct c                 51


<210>  10
<211>  57
<212>  DNA
<213>  Artificial Sequence

<220>
<223>  synthetic oligonucleotide

<400>  10
cgtaacgcgt tcattcaaga gcttaaggac gatccttcag tgagcaaaga gatcctg           57


<210>  11
<211>  60
<212>  DNA
<213>  Artificial Sequence

<220>
<223>  synthetic oligonucleotide

<400>  11
gatgaattct tattttggcg cctgagcatc gttaagcttt ttagcttctg ccaggatctc        60


<210>  12
<211>  174
<212>  DNA
<213>  Artificial Sequence

<220>
<223>  Code DNA

<400>  12
gctgacaaca agtttaacaa agaacaacaa aatgctttct atgagatcct gcatttacct        60

aacttaactg aggaacaacg taacgcgttc attcaagagc ttaaggacga tccttcagtg       120

agcaaagaga tcctggcaga agctaaaaag cttaacgatg ctgaggcgcc aaaa          174

<210>  13
<211>  60
<212>  DNA
<213>  Artificial Sequence

<220>
<223>  synthetic oligonucleotide

<400>  13
gatgaattct tattttggcg cctcagcatc gttaagcttt ttagcttctg ccaggatctc      60

<210>  14
<211>  174
<212>  DNA
<213>  Artificial Sequence

<220>
<223>  Code DNA

<400>  14
gctgacaaca agtttaacga agaacaacaa aatgctttct atgagatcct gcatttacct      60

aacttaactg aggaacaacg taacgcgttc attcaagagc ttaaggacga tccttcagtg     120

agcaaagaga tcctggaaga agctaaaaag cttaacgatg ctcaggcgcc aaaa          174

<210>  15
<211>  60
<212>  DNA
<213>  Artificial Sequence

<220>
<223>  synthetic oligonucleotide

<400>  15
cgtggatccg ctgacaacaa gtttaacgaa gaacaacaaa atgctttcta tgagatcctg      60

<210>  16
<211>  60
<212>  DNA
<213>  Artificial Sequence

<220>
<223>  synthetic oligonucleotide

<400>  16
gatgaattct tattttggcg cctgagcatc gttaagcttt ttagcttctt ccaggatctc      60

<210>  17
<211>  174
<212>  DNA
<213>  Artificial Sequence

<220>
<223>  Code DNA

<400>  17
gctgacaaca agtttaacga agaacaacaa aatgctttct atgagatcct ggatttacct      60

aacttaactg aggaacaacg taacgcgttc attcaagagc ttaaggacga tccttcagac     120

agcaaagaga tcctggacga agctaaaaag cttaacgatg ctgaggcgcc aaaa           174


<210>  18
<211>  51
<212>  DNA
<213>  Artificial Sequence

<220>
<223>  synthetic oligonucleotide

<400>  18
aatgaacgcg ttacgttgtt cctcagttaa gttaggtaaa tccaggatct c               51


<210>  19
<211>  57
<212>  DNA
<213>  Artificial Sequence

<220>
<223>  synthetic oligonucleotide

<400>  19
cgtaacgcgt tcattcaaga gcttaaggac gatccttcag acagcaaaga gatcctg         57


<210>  20
<211>  60
<212>  DNA
<213>  Artificial Sequence

<220>
<223>  synthetic oligonucleotide

<400>  20
gatgaattct tattttggcg cctcagcatc gttaagcttt ttagcttcgt ccaggatctc      60